# EUROPÄISCHE PATENTANMELDUNG

(11) **EP 1 364 613 A1**
(43) Veröffentlichungstag der Anmeldung: **26.11.2003**
(21) Anmeldenummer: 02011322.1
(22) Anmeldetag: 23.05.2002
(51) Int. Cl.: A61B 5/00, A61B 5/0205

(54) **Vorrichtung zur Gesundheitsüberprüfung und -überwachung**

(71) Anmelder: FESTO AG & Co, 73734 Esslingen (DE)
(72) Erfinder: Stoll, Wilfried, Dr. rer. pol., 70184 Stuttgart (DE); Scheurenbrand, Hans, Prof. Dr.-Ing., 71394 Kernen (DE); Schwarz, Michael, 73776 Altbach (DE); Eisenbarth, Gerhard, 71394 Kernen (DE); Walz, Matthias, 73773 Aichwald (DE)
(74) Vertreter: Vetter, Hans, Dipl.-Phys. Dr.

(57) **Zusammenfassung**

Es wird eine Vorrichtung zur Gesundheitsüberprüfung und -überwachung vorgeschlagen, die eine einem einzelnen Patienten zugeordnete Diagnoseeinrichtung (10) besitzt, welche zum Empfang und zur Auswertung von Messdaten einer Vielzahl von Messwertaufnehmern in Messgeräten (11, 15 - 21) für chemische und physikalische Parameter ausgebildet ist. Die Messwertaufnehmer sind nach Art eines Baukastensystems individuell auswählbar und einsetzbar. Ein Display (22) dient zur Wiedergabe der Messdaten und/oder aus allen Messdaten der jeweils vorgesehenen Messwertaufnehmer gewonnenen Diagnosedaten und/oder Warninformationen zur Erkennung eines kritischen Gesundheitszustands. Weiterhin sind Mittel (14) zur Übertragung der Messdaten und/oder Diagnosedaten und/oder Warninformationen an eine Experten-Zentralstelle vorgesehen. Durch das Baukastensystem kann die Vorrichtung individuell an einen einzelnen Patienten angepasst werden, der hinsichtlich einer bestimmten Krankheit oder eines bestimmten Risikos oder des allgemeinen Gesundheitszustands überprüft oder überwacht werden soll.

## Beschreibung

Die Erfindung betrifft eine Vorrichtung zur Gesundheitsüberprüfung und -überwachung, die vom Patienten selbst bedient werden kann.

Aus der Zeitschrift "com!online" 2/2002, S. 16, ist ein Gerät bekannt, das der Patient am Handgelenk trägt und das zur Blutdruckmessung bestimmt ist. Die Messungen erfolgen vollautomatisch, und anschließend werden die Daten über ein Handy an ein Service-Center gesandt, wo Ärzte die Daten auswerten und dem behandelnden Arzt des Patienten zur Verfügung stellen.

Der Anwendungsbereich des bekannten Geräts ist allerdings sehr eingeschränkt, da nur Blutdruckmessungen vorgesehen sind, die selbstverständlich nicht ausreichen, um ein einigermaßen ausreichendes Bild über den Gesundheitszustand des Patienten zu geben, weder für ihn selbst noch für das Service-Center.

Bei physiologischen Untersuchungen, beispielsweise Blut- oder Urinuntersuchungen, ist es üblich, dass der behandelnde Arzt eine Probe nimmt und per Kurier an das Untersuchungslabor überstellt, wo die entsprechenden Tests durchgeführt werden. Die Testergebnisse werden dem behandelnden Arzt meist postalisch, in dringenden Fällen auch telefonisch mitgeteilt. Anhand dieser Testergebnisse kann der behandelnde Arzt die Diagnose stellen und geeignete Maßnahmen zur Therapie einleiten. Diese bekannte Praxis ist umständlich und zeit- und kostenaufwendig.

Eine Aufgabe der vorliegenden Erfindung besteht darin, eine Vorrichtung zur Gesundheitsüberprüfung und -überwachung zu schaffen, bei der der Patient weitgehend selbst alle für eine sichere Diagnose notwendigen Untersuchungen selbst vornimmt oder durch die Vorrichtung automatisch vornehmen lassen kann, ohne dass hierzu die direkte Mithilfe eines Arztes notwendig wäre.

Diese Aufgabe wird erfindungsgemäß durch eine Vorrichtung mit den Merkmalen des Anspruchs 1 gelöst.

Die Vorteile der erfindungsgemäßen Vorrichtung bestehen insbesondere darin, dass mit einem Typus von Diagnoseeinrichtung eine Vielzahl unterschiedlicher Gesundheitsüberprüfungen und -überwachungen durchgeführt werden kann, da die Messwertaufnehmer nach Art eines Baukastensystems individuell auswählbar sind. Diese Auswahl kann beispielsweise durch einen Arzt getroffen werden, der in Abhängigkeit des jeweiligen Gesundheitsrisikos oder der jeweils zu überwachenden oder zu diagnostizierenden Krankheit des Patienten die dafür erforderlichen Messungen physikalischer und chemischer bzw. biochemischer Art festlegen kann. Auch wenn sich das Krankheitsbild oder das Gesundheitsrisiko ändern sollte, so kann dieselbe Diagnoseeinrichtung weiterverwendet werden, und es müssen lediglich einzelne Messwertaufnehmer ausgetauscht werden. Insbesondere bei schleichenden organischen Erkrankungen und zur Therapieüberwachung vor allem bei schweren, chronischen Leiden kann eine Langzeitdiagnose auf einfache Weise erzielt werden. Das Testergebnis wird dem Anwender dann auf dem Display angezeigt, und die Daten werden an ein Service-Center bzw. an eine Experten-Zentralstelle übertragen, um dort von Fachleuten, insbesondere Ärzten, überprüft zu werden. Dabei kann die Diagnoseeinrichtung aus den gewonnenen Messdaten in vorteilhafter Weise zumindest einfache Diagnosen erstellen und dem Patienten bzw. der Zentralstelle mitteilen. Diese Diagnose kann im einfachsten Falle aus einer Warninformation zur Erkennung eines kritischen Gesundheitszustands bestehen. Alternativ oder zusätzlich können auch die Messdaten selbst wiedergegeben werden, wobei kritische Werte bzw. die Überoder Unterschreitung von kritischen Werten entsprechend angezeigt wird. Das Steuerprogramm der Diagnoseeinrichtung kann dabei von sich aus in Abhängigkeit des erkannten Krankheitsbildes Vorschläge zur Ergänzung durch zusätzliche Messwertaufnehmer machen. Dabei kann die Diagnoseeinrichtung auch als lernendes System ausgebildet sein.

Durch die in den Unteransprüchen aufgeführten Maßnahmen sind vorteilhafte Weiterbildungen und Verbesserungen der im Anspruch 1 angegebenen Vorrichtung möglich.

Die Messwertübertragung zwischen den Messwertaufnehmern und der Diagnoseeinrichtung erfolgt vorzugsweise drahtlos, kann jedoch in einer einfacheren Ausführung auch wenigstens teilweise drahtgebunden erfolgen, vorzugsweise dann, wenn die Diagnoseeinrichtung und die entsprechenden Messwertaufnehmer am Körper getragen werden.

In vorteilhafter Weise ist eine Messstation für chemische Parameter vorgesehen, die zur Aufnahme von Blut und/oder Urin und/oder Gewebeproben und/oder Sputum enthaltende Diagnose-Kartuschen ausgebildet ist, wobei die Diagnose-Kartuschen vorzugsweise Einweg-Kartuschen sind. Hierdurch kann der eingangs beschriebene, bisher sehr umständliche physiologische Testablauf wesentlich vereinfacht und durch den Patienten selbst durchgeführt werden.

Die in der Messstation und/oder anderen Messwertaufnehmern enthaltenen Messgeräte sind zweckmäßigerweise an die Diagnoseeinrichtung ankoppelbar oder einstückig mit dieser verbunden oder als separate, örtlich getrennte Station ausgebildet. Hierdurch wird der Einsatz der Vorrichtung und die Anpassung an individuelle Wünsche oder Gegebenheiten noch optimiert.

Die Messwertaufnehmer in der Messstation sind zur Erfassung wenigstens einer der Messwerte Cholesterin, Lipoprotein a, Eiweiß, Laktat, Troponin T, Natrium, Kalium, Calcium, Harnsäure, Ostase, Phosphat, Blutbild, Creatinin ausgebildet, wobei die Diagnose-Kartuschen vorzugsweise eine die Messspezifizierung vorgebende Codierung aufweisen, damit die Messstation erkennt, welcher Test durchgeführt werden soll. Diese Erkennung kann auch durch die Diagnoseeinrichtung erfolgen, die mit der Messstation dann in bidirektionaler Verbindung steht.

Die jeweiligen Messwertaufnehmer für physikalische Parameter sind zweckmäßigerweise in wenigstens einem der folgenden Messgeräte enthalten: Blutdruckmessgerät, Pulsmessgerät, Körperfettwaage, EKG-Gerät, Temperaturmessgerät, Atemluft-Messgerät, Hautanalyse-Messgerät, Glukose-/Laktat-Messgerät. Diese Messgeräte stehen vorzugsweise mit der Diagnoseeinrichtung in drahtloser Verbindung zur Messwertübertragung.

Die Diagnoseeinrichtung kann als PC oder Laptop oder als am Körper des Patienten tragbares Gerät ausgebildet sein. Vor allem zur kontinuierlichen Überwachung des Patienten bzw. dessen Gesundheitszustands oder für Langzeituntersuchungen eignet sich ein am Körper des Patienten tragbares Gerät. Dabei ist vorzugsweise auch wenigstens ein Teil der die Messwertaufnehmer enthaltenden Messgeräte am Körper des Patienten tragbar ausgebildet.

Die am Körper des Patienten tragbar ausgebildete Diagnoseeinrichtung ist in vorteilhafter Weise mit einem GPS-System zur Ortsbestimmung des Patienten versehen, wobei die GPS-Daten ständig oder zyklisch neben den Mess- und/oder Diagnosedaten der Experten-Zentralstelle übermittelt werden. Bei akutem Notfall, der aus den übermittelten Daten in der Experten-Zentralstelle erkannt wird, kann der Patient lokalisiert und es können Notfallmaßnahmen eingeleitet werden.

Die Diagnoseeinrichtung besitzt in vorteilhafter Weise Mittel zur Vorgabe von Therapievorschlägen in Abhängigkeit der Messund/oder Diagnosedaten und/oder Mittel zur Kommunikation mit dem Patienten und/oder zur Vorgabe und Änderung des Prüf- und Überwachungsvorgangs.

Die Diagnoseeinrichtung ist zweckmäßigerweise mit einem Batterieladegerät versehen oder an eine mit einem Batterieladegerät versehene Docking-Station ankoppelbar, die vorzugsweise auch die Mittel zur Übertragung der Messdaten und/oder Diagnosedaten und/oder Warnfunktionen enthält. Hierdurch kann die Diagnoseeinrichtung selbst besonders kleinvolumig und leicht ausgebildet sein.

Die Mittel zur Übertragung der Messdaten und/oder Diagnosedaten und/oder Warnfunktion sind vorzugsweise als Internet-Schnittstelle und/oder Telefon-Schnittstelle für drahtlose oder drahtgebundene Datenübertragung ausgebildet. Diese Mittel können in der Diagnoseeinrichtung selbst oder in der Docking-Station vorgesehen sein.

In vorteilhafter Weise ist ein dem Patienten zugeordnetes Identifikationsmittel, insbesondere eine Identifikationskarte, vorgesehen, durch die die Diagnoseeinrichtung und/oder die Messstation für chemische Parameter und/oder wenigstens ein weiteres Messgerät auf den Patienten konfigurierbar ist. Diese Konfiguration kann beispielsweise von einem Arzt oder der Experten-Zentralstelle vorgenommen werden, so dass Fehlbedienungen ausgeschlossen sind. Durch solche Identifikationsmittel kann eine Diagnoseeinrichtung auch von mehreren Patienten benutzt werden, die jeweils beispielsweise durch Einstecken der Identifikationskarte ihre persönliche Konfiguration der Diagnoseeinrichtung vornehmen können.

Ausführungsbeispiele der Erfindung sind in der Zeichnung dargestellt und in der nachfolgenden Beschreibung näher erläutert. Es zeigen:

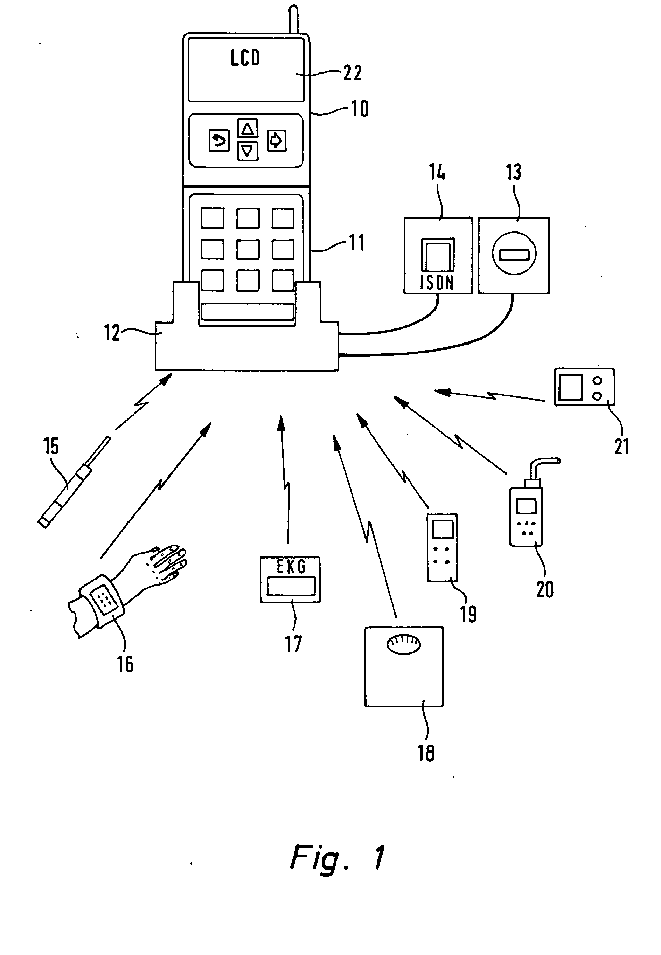

- Figur 1: ein erstes Ausführungsbeispiel der Erfindung, bei dem eine tragbare Diagnoseeinrichtung an eine Docking-Station angekoppelt ist und mit mehreren Messgeräten zum Datenempfang in drahtloser Verbindung steht, und
- Figur 2: ein zweites Ausführungsbeispiel mit einer als Laptop ausgebildeten Diagnoseeinrichtung, die mit einer Messstation für chemische Parameter in drahtloser Verbindung steht.

Bei dem in Figur dargestellten Ausführungsbeispiel ist eine Diagnoseeinrichtung 10 mit einer Messstation 11 für chemische bzw. biochemische Parameter lösbar verbunden und bildet ein Gerät, das in eine Docking-Station 12 eingesteckt oder an dieser angekoppelt ist. Die Docking-Station 12 enthält ein nicht näher dargestelltes Batterieladegerät für in der Diagnoseeinrichtung 10 und/oder der Messstation 11 angeordnete Batterien und ist hierzu mit einer Netzsteckdose 13 verbindbar. Weiterhin besitzt die Docking-Station 12 ein ISDN-Modem für die Datenübertragung von der Diagnoseeinrichtung 10 zu einer Experten-Zentralstelle über das Internet. Die Docking-Station 12 ist hierzu mit einer ISDN-Steckdose 14 verbindbar.

Anstelle eines ISDN-Modems kann selbstverständlich auch eine andere Schnittstelle für das Internet vorgesehen sein, beispielsweise ein DSL- oder analoges Modem. Weiterhin kann die Datenübertragung auch direkt per Funk erfolgen. Hierzu wäre dann ein Funktelefon in oder an der Diagnoseeinrichtung 10 angeordnet oder mit dieser verbindbar.

Die Diagnoseeinrichtung 10 ist zum Datenempfang von Messdaten einer Vielzahl von Messgeräten ausgebildet. Diese Messgeräte sind im Ausführungsbeispiel schematisch dargestellt. Es handelt sich dabei im Einzelnen um ein Hautanalyse-Messgerät 15, ein kombiniertes Blutdruck- und Pulsmessgerät 16, ein EKG-Messgerät 17, eine Körperfettwaage 18, ein Sauerstoff-Messgerät 19, ein Atemluft-Messgerät 20 und ein Glukose-/Laktat-Messgerät 21. Diese Messgeräte stehen mit der Diagnoseeinrichtung 10 in drahtloser Verbindung, beispielsweise über Funk oder Infrarot, sie können jedoch prinzipiell auch die Daten über Leitungen übertragen, insbesondere dann, wenn die Messgeräte am Körper des Patienten getragen werden, wie beispielsweise das Blutdruck- und Pulsmessgerät 16. Dieses könnte auch in zwei separate Geräte aufgeteilt sein. Weiterhin kann dieses Messgerät 16 oder ein anderes, am Körper getragenes Messgerät auch noch die Körpertemperatur des Patienten bestimmen.

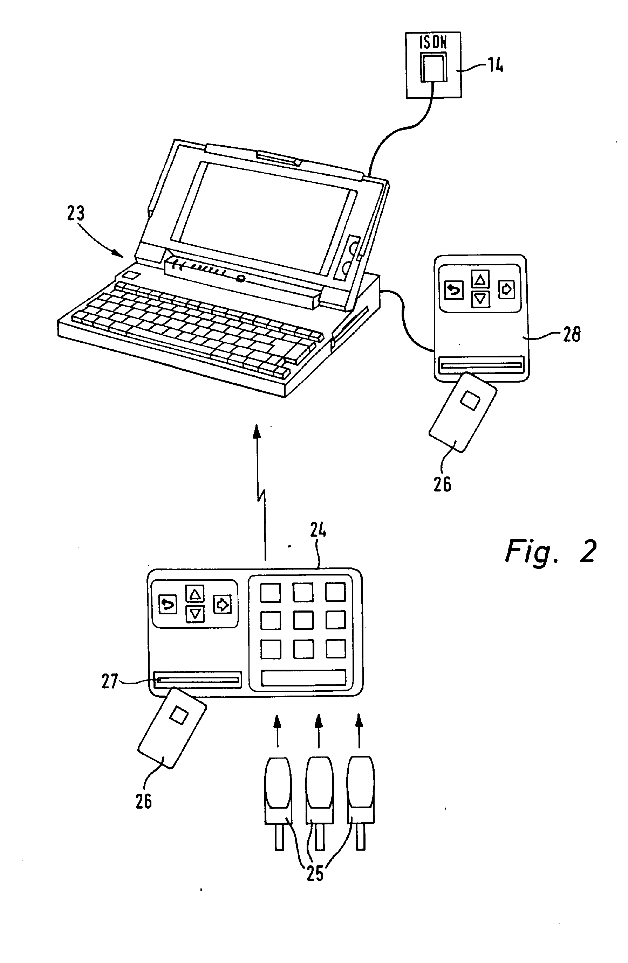

Die Messstation 11 für chemische bzw. biochemische Parameter, die entweder fest oder lösbar an der Diagnoseeinrichtung 10 angebracht ist, beispielsweise ansteckbar, kann selbstverständlich auch als separate Messstation ausgebildet sein, die ihre Messdaten wie die anderen Messgeräte drahtlos oder drahtgebunden der Diagnoseeinrichtung 10 übermittelt. Diese Messstation dient zur Erfassung wenigstens eines der Messwerte Cholesterin, Lipoprotein a, Eiweiß, Laktat, Troponin T, Natrium, Kalium, Calcium, Harnsäure, Ostase, Phosphat, Blutbild, Creatinin anhand von eingesetzten Blut-, Urin-, Sputumund/oder Gewebeproben. Dies wird in Verbindung mit Figur 2 noch näher erläutert.

Die Diagnoseeinrichtung 10 ist quasi das Gehirn der gesamten Vorrichtung und ist in Prozessortechnik ausgeführt, das heißt, sie enthält wenigstens einen Mikrocontroller. In dieser Diagnoseeinrichtung werden die erfassten Messwerte verarbeitet und an einen externen Speicher übermittelt oder intern gespeichert. Die wichtigsten Vitalparameter können online überwacht werden. Diese Diagnoseeinrichtung ist als Universalgerät ausgebildet und fähig, die Vielzahl der verschiedenen chemischen und physikalischen Parameter zu erfassen und zu verarbeiten, wobei die tatsächlich eingesetzten Messgeräte nach Art eines Baukastensystems individuell ausgewählt und eingesetzt werden. Die Auswahl erfolgt beispielsweise durch den Arzt in Abhängigkeit der zu überwachenden Risiken bzw. Krankheiten. Nach erfolgter Auswahl wird der Patient in die Lage versetzt, vom Programm der Diagnoseeinrichtung geleitet die notwendigen Tests selbst auszuwählen und diese bei sich zu Hause durchzuführen. Die Messwerte und die Testergebnisse werden dem Anwender auf dem Display 22 der Diagnoseeinrichtung 10 optisch angezeigt und zugleich in der beschriebenen Weise der Experten-Zentralstelle oder einem Arzt übermittelt. Der Arzt hat auch die Möglichkeit, die gewonnenen Daten, die zentral oder dezentral beim Patienten gespeichert sind, einzusehen und für seine eigene Diagnose heranzuziehen. Durch die Ablage der Daten aller Tests können auch Zeitverläufe wichtiger Parameter erstellt werden, was bei der medikamentösen Einstellung chronischer Krankheiten besonders hilfreich ist. Die Diagnoseeinrichtung 10 kann auch eine Interpretation der gewonnenen Messwerte vornehmen und daraus eine Diagnose und sogar einen Vorschlag für eine Therapie erstellen. Die Diagnoseeinrichtung 10 kann durch ihr Programm in die Lage versetzt werden, den Patienten mit der Zeit besser einzuschätzen, so dass einzelne Testergebnisse durch den Vergleich mit früheren Ergebnissen oder den Werten anderer Tests deutlich an Aussagekraft gewinnen. Dies kann so weit gehen, dass die Diagnoseeinrichtung 10 dem Anwender das Gesundheits-Management organisieren kann.

Die Diagnoseeinrichtung 10 kann auch mit dem Patienten kommunizieren und Zustände abfragen, beispielsweise Schmerzzustände, Übelkeit, Schweißausbrüche und dergleichen, die der Patient dann über Bedienungselemente der Diagnoseeinrichtung 10 eingeben kann. Durch diese Abfragen können die für die Diagnose erforderlichen Daten noch ergänzt werden.

Da die Diagnoseeinrichtung 10 über Funk, Telefon oder Internet mit der Experten-Zentralstelle bzw. einem Arzt in Verbindung steht, kann sie auch als mobiles Überwachungsgerät dienen und bei Bedarf selbstständig die Notfallversorgung anordnen und einleiten. Hierzu kann sie ein Ortungssystem, beispielsweise ein GPS-System, enthalten, um den Standort des Notfallpatienten zu ermitteln und zu übermitteln.

Die Ergebnisse von Studien über bestimmte Krankheiten, Krankheitsverläufe, Diagnosen und Indikationen können direkt oder über die beschriebenen Verbindungssysteme, wie Funk oder Internet, in die Diagnoseeinrichtung 10 eingespeist oder von dieser abgefragt werden. In Verbindung mit den beispielsweise anonymisierten Daten mehrerer Anwender oder eines Anwenders entsteht eine Datenbasis, aus welcher der Einfluss der einzelnen Parameter auf den Gesundheitszustand präzise ermittelt werden kann. Die Diagnoseeinrichtung 10 kann so ausgestattet zum Teil die Aufgaben des Arztes übernehmen und anhand gezielter Fragen - wie bereits beschrieben - eine präzise Anamnese durchführen. Durch die gewonnenen Informationen über die Beschwerden des Anwenders und die Kenntnis seiner Vorgeschichte ist die Diagnoseeinrichtung 10 auch in der Lage, zu entscheiden, welche Parameter überprüft werden müssen, um die richtige Diagnose zu stellen bzw. einen Verdacht auszuschließen. Falls diese Parameter bisher noch nicht überprüft werden, so fordert die Diagnoseeinrichtung 10 den Patienten auf, dafür zu sorgen, dass entsprechende zusätzliche Messgeräte oder zusätzliche chemische bzw. biochemische Untersuchungen in das System integriert werden. Die durch die Diagnoseeinrichtung 10 erstellte Diagnose und die erstellten Therapievorschläge sind optimal auf den jeweiligen Patienten bzw. Anwender zugeschnitten. Dabei können auch Ereignisse früherer Therapien, wie zum Beispiel die persönliche Verträglichkeit bestimmter Medikamente oder allergische Reaktionen gegen einzelne Wirkstoffe, berücksichtigt werden. Natürlich kann der Patient über die Vernetzung in jeder Phase der Behandlung einen Arzt zu Rate ziehen und die Vorschläge der Diagnoseeinrichtung 10 überprüfen lassen. Neben der Begleitung von Untersuchung und Therapie kann die Diagnoseeinrichtung 10 ein auf die persönlichen Gesundheitsrisiken des Patienten abgestimmtes Programm zur Früherkennung von Krankheiten erstellen und den Anwender zu gegebener Zeit auf die Notwendigkeit einer Untersuchung hinweisen.

Bei dem in Figur 2 dargestellten zweiten Ausführungsbeispiel ist eine als Laptop ausgebildete Diagnoseeinrichtung 23 vorgesehen. Anstelle eines Laptops kann auch ein üblicher PC oder ein anderer Computer treten. Die Funktionen entsprechen prinzipiell denen der bereits beschriebenen Diagnoseeinrichtung 10. Das heißt, auch die Diagnoseeinrichtung 23 ist zum drahtlosen oder drahtgebundenen Empfang einer Vielzahl von Messwerten ausgebildet, beispielsweise der Messwerte der Messgeräte 15 - 21, die zur Vereinfachung nicht nochmals dargestellt sind. Anstelle der mit der Diagnoseeinrichtung 10 verbundenen Messstation 11 für chemische bzw. biochemische Parameter tritt eine separate Messstation 24 für solche chemischen bzw. biochemischen Parameter, die mit der Diagnoseeinrichtung 23 zur Übermittlung der Messwerte in drahtloser Verbindung steht, wobei auch eine drahtgebundene Übermittlung möglich ist. Die Funktion der Messstation 24 entspricht im Wesentlichen der der Messstation 11. Sie besitzt eine Einrichtung zur Aufnahme von mit Proben versehehen Diagnose-Kartuschen 25. Diese Diagnose-Kartuschen 25 sind beispielsweise als Einweg-Kartuschen ausgebildet und dienen zur Aufnahme von Proben, wie Blutproben, Urinproben, Sputumproben oder Gewebeproben. Die Diagnose-Kartuschen 25 sind codiert, beispielsweise durch eine mechanische, magnetische oder elektromagnetische Codierung, so dass die Messstation 24 oder die Diagnoseeinrichtung 23 erkennt, welcher Test durchgeführt werden soll, und die gesamte Testdurchführung entsprechend steuern kann. Die Messstation 24 stellt dabei das ausführende Organ dar.

Zur Durchführung der Tests besitzt die Messstation 24 die dafür notwendigen Messwertaufnehmer, wobei mikrofluidische und mikrooptische Elemente auch in den Diagnose-Kartuschen 25 integriert sein können. Die Messwertaufnahme erfolgt beispielsweise in Form von optischen Komponenten, zum Beispiel Bereitstellung von polarisiertem Licht der für den Test notwendigen Wellenlänge und dem dazugehörenden Sensor zur Messung der Intensität des Lichtstrahls nach Durchdringung der aufbereiteten Probe in der Diagnose-Kartusche 25. Andere bekannte Testverfahren zur Blutuntersuchung, Urinuntersuchung, Sputumuntersuchung und der Untersuchung von Gewebeproben können ebenfalls in der Messstation 24 enthalten sein. Die Diagnose-Kartuschen 25 können jeweils für spezielle Tests individuell ausgestaltet sein oder auch für verschiedene Tests geeignet sein. In diesem Falle muss der Diagnoseeinrichtung 23 oder der Messstation 24 durch entsprechende Eingaben die Art der gewünschten Messung mitgeteilt werden. Dies kann bei der Einstellung der Diagnoseeinrichtung 23 erfolgen. Die Einstellung der Diagnoseeinrichtung 23 und/oder der Messstation 24 kann auch durch Codier-Elemente, beispielsweise Codier-Karten 26, erfolgen. Hierzu besitzt die Messstation 24 eine entsprechende Steckaufnahme 27, und die Diagnoseeinrichtung 23 ist mit einer Kartenleseeinrichtung 28 verbunden oder enthält eine solche. Die Codier-Karte 26 kann vom Arzt oder der Experten-Zentralstelle codiert und auf den speziellen Patienten abgestimmt werden. Die Codierung bestimmt die Art und den Umfang der Messungen, wobei zusätzliche Informationen über den Patienten enthalten sein können. Alternativ hierzu kann die Codierung der Diagnoseeinrichtung 23 bzw. der Messstation 24 vom Arzt oder der Experten-Zentralstelle direkt oder über die beschriebenen Kommunikationsmittel, wie Funk oder Internet, erfolgen. Durch die Codierung, insbesondere die Codier-Karten 26, können auch verschiedene Patienten dieselbe Diagnoseeinrichtung 23 und dieselbe Messstation 24 benützen, wobei zur Einstellung jeweils nur die entsprechende Codier-Karte 26 eingesteckt zu werden braucht. Die mit den Messstationen 24 bzw. 1 durchzuführenden Tests können zum Beispiel zur Ermittlung der Werte für Cholesterin, Lipoprotein a, Eiweiß, Laktat, Troponin T, Natrium, Kalium, Calcium, Harnsäure, Ostase, Phosphat, Blutbild, Creatinin und dergleichen dienen. Je nach Test enthält die jeweilige Kartusche mehr oder wenige komplexe mikrofluidische Strukturen, zum Beispiel Ventile, Pumpen, Filter und dergleichen, in denen die gesamte Probenaufbereitung automatisch durchgeführt werden kann. Auch die Stelle, an der die Messung erfolgt, ist in dem System in Form von zum Beispiel optisch durchlässigen Stellen integriert, wie dies bereits erläutert wurde. Die Diagnose-Kartuschen für die verschiedenen Tests besitzen ein einheitliches Design, so dass mit einer Messstation 11 bzw. 24 viele verschiedene Tests durchgeführt werden können. Selbstverständlich können in speziellen Fällen auch mehrere Messstationen vorgesehen sein.

In bestimmten Fällen kann eine Fehlfunktion des Körpers nicht durch einen Test allein festgestellt werden. Dies gilt zum Beispiel für die Abschätzung des Arteriosklerose-Risikos. Um das Risiko im Einzelnen einschätzen zu können, sind verschiedene Parameter, zum Beispiel Apolipoprotein A, Apolipoprotein B, Cholesterin, Homocystein, Lipoprotein A und die Triglyceride, zu messen. Es können daher bestimmte Diagnose-Kartuschen 25 so ausgebildet sein, dass sie alle diese Parameter für eine bestimmte Untersuchung messen können. Damit kann die Dauer der Untersuchung wesentlich reduziert werden und senkt die Belastung des Patienten, der dann beispielsweise nicht alle 10 Minuten eine Probe nehmen muss, was auch die Akzeptanz wesentlich erhöht. Neben den chemischen bzw. biochemischen Parametern müssen auch häufig physikalische Parameter bestimmt werden, so ist für die Abschätzung des Arteriosklerose-Risikos beispielsweise noch die Erfassung des Blutdrucks erforderlich.

Zur Überwachung oder Überprüfung des Allgemeinzustands können beispielsweise in der Messstation 11 bzw. 24 die Werte für Cholesterin, Eiweiß, Harnsäure und das Blutbild überprüft werden, während gleichzeitig als physikalische Parameter der Blutdruck und das EKG bestimmt werden.

Als Diagnose kann durch die Diagnoseeinrichtung 10 bzw. 23 ein bestimmter Verdacht, eine Risikoziffer, Gesundheitstipps und/oder eine detaillierte Diagnose wiedergegeben bzw. übertragen werden.

## Patentansprüche

1. Vorrichtung zur Gesundheitsüberprüfung und -überwachung, mit einer einem einzelnen Patienten zugeordneten Diagnoseeinrichtung (10; 23), die zum Empfang und zur Auswertung von Messdaten einer Vielzahl von Messwertaufnehmern für chemische und physikalische Parameter ausgebildet ist, wobei die Messwertaufnehmer nach Art eines Baukastensystems individuell auswählbar und einsetzbar sind, mit einem Display (22) zur Wiedergabe der Messdaten und/oder aus allen Messdaten der jeweils vorgesehenen Messwertaufnehmer gewonnenen Diagnosedaten und/oder Warninformationen zur Erkennung eines kritischen Gesundheitszustands und mit Mitteln (14) zur Übertragung der Messdaten und/oder der Diagnosedaten und/oder Warninformationen an eine Experten-Zentralstelle.

2. Vorrichtung nach Anspruch 1, **dadurch gekennzeichnet, dass** eine drahtlose und/oder drahtgebundene Messwertübertragung zwischen Messwertaufnehmern und Diagnoseeinrichtung (10; 23) vorgesehen ist.

3. Vorrichtung nach Anspruch 1 oder 2, **dadurch gekennzeichnet, dass** eine Messstation (11; 24) für chemische Parameter vorgesehen ist, die zur Aufnahme von Blut und/oder Urin und/oder Gewebeproben und/oder Sputum enthaltende Diagnose-Kartuschen (25) ausgebildet ist, wobei die Diagnose-Kartuschen (25) vorzugsweise Einwe-Kartuschen sind.

4. Vorrichtung nach Anspruch 3, **dadurch gekennzeichnet, dass** die Messstation (11; 24) und/oder andere Messwertaufnehmer enthaltende Messgeräte an die Diagnoseeinrichtung (10; 23) ankoppelbar oder einstückig mit dieser verbunden ist oder als separate, örtlich getrennte Station ausgebildet ist.

5. Vorrichtung nach einem der vorhergehenden Ansprüche, **dadurch gekennzeichnet, dass** die Messwertaufnehmer in der Messstation (11; 24) zur Erfassung wenigstens einer der Messwerte Cholesterin, Lipoprotein a, Eiweiß, Laktat, Troponin T, Natrium, Kalium, Calcium, Harnsäure, Ostase, Phosphat, Blutbild, Creatinin ausgebildet sind, wobei die Diagnose-Kartuschen (25) vorzugsweise eine die Messspezifizierung vorgebende Codierung aufweisen.

6. Vorrichtung nach einem der vorhergehenden Ansprüche, **dadurch gekennzeichnet, dass** die jeweiligen Messwertaufnehmer für physikalische Parameter in wenigstens einem der folgenden Messgeräte enthalten sind: Hautanalyse-Messgerät (15), Blutdruckmessgerät und Pulsmessgerät (16), EKG-Messgerät (17). Körperfettwaage (18), Sauerstoff-Messgerät (19), Atemluft-Messgerät (20), Glukose-/Laktat-Messgerät (21), Temperatur-Messgerät.

7. Vorrichtung nach einem der vorhergehenden Ansprüche, **dadurch gekennzeichnet, dass** die Diagnoseeinrichtung (10; 23) als PC oder Laptop oder als am Körper des Patienten tragbares Gerät ausgebildet ist.

8. Vorrichtung nach einem der vorhergehenden Ansprüche, **dadurch gekennzeichnet, dass** wenigstens ein Teil der die Messwertaufnehmer enthaltenden Messgeräte (15 - 21) am Körper des Patienten tragbar ausgebildet ist.

9. Vorrichtung nach einem der vorhergehenden Ansprüche, **dadurch gekennzeichnet, dass** die am Körper des Patienten tragbare Diagnoseeinrichtung (10; 23) mit einer Vorrichtung zur Ortsbestimmung des Patienten, insbesondere GPS-System, versehen ist, und die Ortsdaten ständig oder zyklisch neben den Mess- und/oder Diagnosedaten der Experten-Zentralstelle übermittelt werden.

10. Vorrichtung nach einem der vorhergehenden Ansprüche, **dadurch gekennzeichnet, dass** die Diagnoseeinrichtung (10; 23) Mittel zur Vorgabe von Therapievorschlägen in Abhängigkeit der Mess- und/oder Diagnosedaten und/oder Mittel zur Kommunikation mit dem Patienten und/oder zur Vorgabe und Änderung des Prüf- und Überwachungsvorgangs besitzt.

11. Vorrichtung nach einem der vorhergehenden Ansprüche, **dadurch gekennzeichnet, dass** die Diagnoseeinrichtung (10; 23) mit einem Batterieladegerät versehen oder an eine mit einem Batterieladegerät versehene Docking-Station (12) ankoppelbar ist, die vorzugsweise auch die Mittel zur Übertragung der Messdaten und/oder Diagnosedaten und/oder Warnfunktionen enthält.

12. Vorrichtung nach einem der vorhergehenden Ansprüche, **dadurch gekennzeichnet, dass** die Mittel zur Übertragung der Messdaten und/oder Diagnosedaten und/oder Warnfunktionen als Internet-Schnittstelle und/oder Telefon-Schnittstelle für drahtlose oder drahtgebundene Datenübertragung ausgebildet sind.

13. Vorrichtung nach einem der vorhergehenden Ansprüche, **dadurch gekennzeichnet, dass** ein dem Patienten zugeordnetes Identifikationsmittel, insbesondere eine Identifikationskarte (26), vorgesehen ist, durch die die Diagnoseeinrichtung (10; 23) und/oder die Messstation (11; 24) für chemische Parameter und/oder wenigstens ein Messgerät (15 - 21) auf den Patienten konfigurierbar ist.
